# EUROPEAN PATENT APPLICATION

(11) **EP 2 540 707 A1**
(43) Date of publication of application: **02.01.2013**
(21) Application number: 11747334.8
(22) Date of filing: 22.02.2011
(51) Int. Cl.: C07D 213/22, C07D 213/26, C07D 213/85, C07D 401/14, C09K 11/06, H01L 51/50

(54) **SUBSTITUTED PYRIDYL COMPOUND AND ORGANIC ELECTROLUMINESCENT ELEMENT**

(30) Priority: 25.02.2010 JP 2010039565
(71) Applicant: Hodogaya Chemical Co., Ltd., Tokyo 105-0011 (JP); Shinshu University, Matsumoto-shi, Nagano 390-8621 (JP)
(72) Inventor: YOKOYAMA Norimasa, Ibaraki (JP); HAYASHI Shuichi, Ibaraki (JP); ICHIKAWA Musubu, Nagano 390-8621 (JP); YAMAMOTO Takayuki, Nagano 390-8621 (JP)
(74) Representative: Müller-Boré & Partner Patentanwälte
(86) International application number: PCT/JP2011/053849
(87) International publication number: WO 2011/105373

(57) **Abstract**

The present invention relates to a substituted pyridyl compound represented by the following general formula (1), (2), or (3) and an organic electroluminescent element containing a pair of electrodes and at least one organic layer interposed therebetween, in which the at least one organic layer contains the substituted pyridyl compound represented by the following general formula (1), (2), or (3).

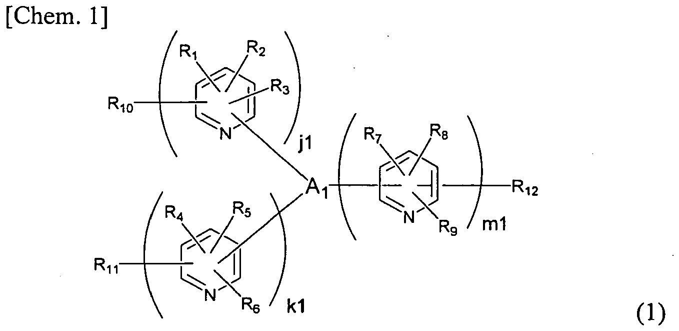

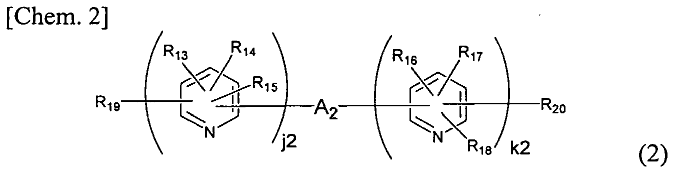

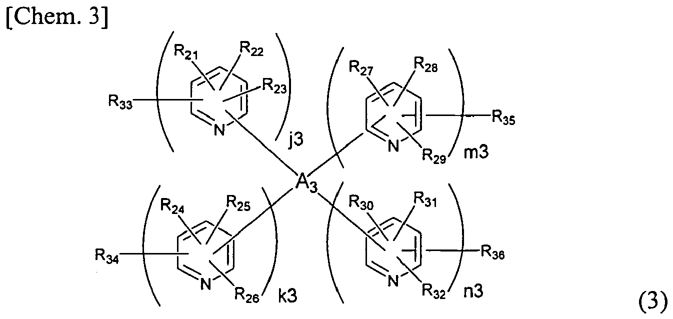

## Description

### TECHNICAL FIELD

The present invention relates to a compound suitable for an organic electroluminescent element which is a self-luminescent element suitable for various displaying devices and an element. More specifically, it relates to a substituted pyridyl compound organic electroluminescent element using the compound.

### BACKGROUND ART

Since organic electroluminescent elements are self-luminescent elements, they are bright and excellent in visibility as compared with liquid-crystalline elements and capable of giving clear display, so that they have been actively studied.

In 1987, C. W. Tang et al. of Eastman Kodak Company put an organic electroluminescent element using organic materials into practical use by developing an element having a multilayered structure in which various roles are assigned to respective materials. Specifically, a fluorescent material capable of transporting electrons and an organic material capable of transporting holes are laminated with each other, so that the both charges are injected into the layer of the fluorescent material to emit light, thereby achieving a high luminance of 1,000 cd/m² or more at a voltage of 10 V or lower (see e.g., Patent Document 1 and Patent Document 2).

To date, many improvements have been performed for practical utilization of the organic electroluminescent elements, and high efficiency and durability have been achieved by an electroluminescent element in which an anode, a hole-injecting layer, a hole-transporting layer, an emitting layer, an electron-transporting layer, an electron-injecting layer, and a cathode are sequentially provided on a substrate, to further segmentalize the various roles (see e.g., Non-Patent Document 1).

Moreover, for the purpose of further improvement of luminous efficiency, utilizaiton of triplet exciton has been attempted and utilization of phosphorescent emitting material has been investigated (see e.g., Non-Patent Document 2).

The emitting layer can be also prepared by doping a charge-transporting compound, generally called a host material, with a fluorescent material or a phosphorescent emitting material. As described in the above-mentioned Non-Patent Documents 1 and 2, the choice of the organic materials in organic electroluminescent elements remarkably affects various properties such as efficiency and durability of the elements.

In the organic electroluminescent elements, the charges injected from the both electrodes are recombined in the emitting layer to attain light emission. However, since the mobility of holes is higher than the mobility of electrons, a problem of reduction in efficiency caused by a part of the holes passing through the emitting layer arises. Therefore, it is required to develop an electron-transporting material in which the mobility of electrons is high.

A representative emitting material, tris(8-hydroxyquinoline)aluminum (hereinafter referred to as Alq₃) is commonly used also as an electron-transporting material, but it cannot be considered that the material has hole-blocking ability.

As a technique to prevent the passing of a part of holes through the emitting layer and to improve probability of charge recombination in the emitting layer, there is a method of inserting a hole-blocking layer. As hole-blocking materials, there have been hitherto proposed triazole derivatives (see e.g., Patent Document 3), bathocuproine (hereinafter referred to as BCP), a mixed ligand complex of aluminum (BAlq) (see e.g., Non-Patent Document 2), and the like.

For example, as an electron-transporting material excellent in hole-blocking ability, there is proposed 3-(4-biphenylyl)-4-phenyl-5-(4-t-butylphenyl)-1,2,4-triazole (hereinafter referred to as TAZ) (see e.g., Patent Document 3).

Since TAZ has a work function as large as 6.6 eV and thus exhibits high hole-blocking ability, it is used as an electron-transportable hole-blocking layer to be laminated onto the cathode side of a fluorescence-emitting layer or phosphorescence-emitting layer prepared by vacuum deposition, coating or the like, and contributes to increase the efficiency of organic electroluminescent elements (see e.g., Non-Patent Document 3).

However, TAZ has a great problem of having low electron transport property, and it is necessary to prepare an organic electroluminescent element in combination with an electron-transporting material having a higher electron transport property (see e.g., Non-Patent Document 4).

Further, BCP has a work function as large as 6.7 eV and high hole-blocking ability, but has a low glass transition point (Tg) of 83°C, so that it is poor in thin-film stability and thus it cannot be considered that it sufficiently functions as a hole-blocking layer. As a technique for lifetime improvement in phosphorescence-emitting elements, there is also proposed to utilize BAlq as a hole-blocking layer. In such element, lifetime improvement is achieved, but holes cannot efficiently be trapped in an emitting layer since BAlq has a small work function of 5.8 eV, reduction in efficiency is observed as compared with an element obtained by utilizing BCP, and therefore it cannot be considered as enough.

All the materials are insufficient in film stability or are insufficient in the function of blocking holes. In order to improve characteristic properties of the organic electroluminescent elements, it is desired to develop an organic compound which is excellent in electron-injection/transport performances and hole-blocking ability and is highly stable in a thin-film state.

### CITATION LIST

### PATENT DOCUMENT

Patent Document 1: JP-A-8-48656
Patent Document 2: Japanese Patent No. 3194657
Patent Document 3: Japanese Patent No. 2734341
Patent Document 4: JP-A-2004-284971

### NON-PATENT DOCUMENT

Non-Patent Document 1: The Japan Society of Applied Physics Ninth Workshop Preprint, pp. 55-61 (2001)
Non-Patent Document 2: The Japan Society of Applied Physics Ninth Workshop Preprint, pp. 23-31 (2001)
Non-Patent Document 3: Fiftieth Meeting of Japan Society of Applied Physics and Related Societies, 28p-A-6 Lecture Preprint, p. 1413 (2003)
Non-Patent Document 4: The Japan Society of Applied Physics, Journal of Molecular Electronics and Bioelectronics section, Vol. 11, No. 1, pp. 13-19 (2000)
Non-Patent Document 5: The Japan Society of Applied Physics, Molecular Electronics and Bioelectronics section Ninth Workshop, pp. 23-31 (2001)
Non-Patent Document 6: J. Org. Chem., 60, 7508 (1995)
Non-Patent Document 7: Synth. Commun., 11, 513 (1981)

### SUMMARY OF INVENTION

### TECHNICAL PROBLEM

Objects of the present invention are to provide an organic compound having excellent properties, which is excellent in electron-injection/transport performances, has hole-blocking ability and has high stability in a thin-film state, as a material for an organic electroluminescent element having high efficiency and high durability, and to provide an organic electroluminescent element having high efficiency and high durability using the compound. As physical properties of the organic compound suitable for the present invention, there may be mentioned (1) good electron injection characteristic, (2) high electron mobility, (3) excellent hole-blocking ability, (4) good stability in a thin-film state, and (5) excellent thermal resistance. In addition, as physical properties of the organic electroluminescent element suitable for the present invention, there may be mentioned (1) high luminous efficiency, (2) low emission initiation voltage, (3) low practical driving voltage.

### MEANS FOR SOLVING THE PROBLEMS

Thus, in order to achieve the above objects, the present inventors have designed and chemically synthesized substituted pyridyl compounds, with focusing on the fact that the nitrogen atom of the pyridine ring which exhibits affinity to an electron has an ability of coordinating to a metal and is excellent in thermal resistance. The present inventors have experimentally produced various organic electroluminescent elements using the compounds, and have extensively performed property evaluation of the elements. As a result, they have accomplished the present invention.

Namely, the present invention provides a substituted pyridyl compound represented by the following general formula (1), (2), or (3); and an organic electroluminescent element containing a pair of electrodes and at least one organic layer interposed therebetween, in which the at least one organic layer contains the substituted pyridyl compound represented by the following general formula (1), (2), or (3).

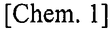

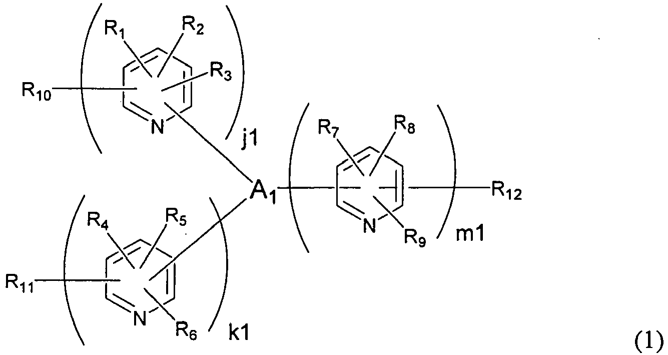

(in the formula, R₁ to R₁₂ may be the same or different and represent a hydrogen atom, a deuterium atom, a fluorine atom, a chlorine atom, a cyano group, a trifluoromethyl group, a linear or branched alkyl group having 1 to 6 carbon atoms which may have a substituent, a substituted or unsubstituted aromatic hydrocarbon group, a substituted or unsubstituted aromatic heterocyclic group, or a substituted or unsubstituted condensed polycyclic aromatic group; j1, k1, and m1 represent an integer of 1 to 4, excluding the case where all of j1, k1, and m1 have an identical value simultaneously; plurality of R₁ to R₉ present in one molecule may be the same or different from one another; and A₁ represents a trivalent group of a substituted or unsubstituted aromatic hydrocarbon, a trivalent group of a substituted or unsubstituted aromatic heterocycle, a trivalent group of a substituted or unsubstituted condensed polycyclic aromatic, or a trivalent group represented by the following general formula (1-1):

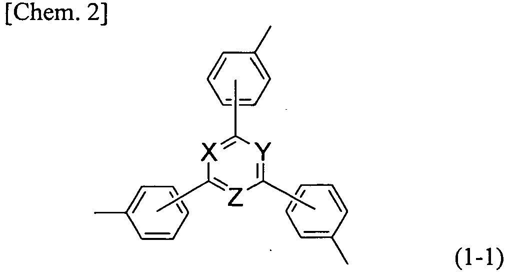

(in the formula, X, Y, and Z represent a carbon atom or a nitrogen atom));

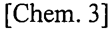

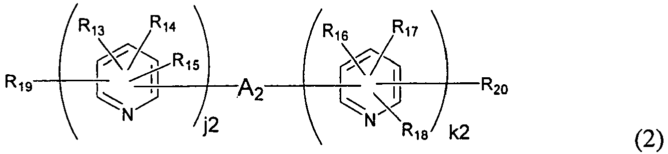

(in the formula, R₁₃ to R₂₀ may be the same or different and represent a hydrogen atom, a deuterium atom, a fluorine atom, a chlorine atom, a cyano group, a trifluoromethyl group, a linear or branched alkyl group having 1 to 6 carbon atoms which may have a substituent, a substituted or unsubstituted aromatic hydrocarbon group, a substituted or unsubstituted aromatic heterocyclic group, or a substituted or unsubstituted condensed polycyclic aromatic group; j2 and k2 represent an integer of 3 to 5, excluding the case where j2 and k2 have an identical value; plurality of R₁₃ to R₁₈ present in one molecule may be the same or different from one another; and A₂ represents a divalent group of a substituted or unsubstituted aromatic hydrocarbon, a divalent group of a substituted or unsubstituted aromatic heterocycle, a divalent group of a substituted or unsubstituted condensed polycyclic aromatic, or a single bond); and

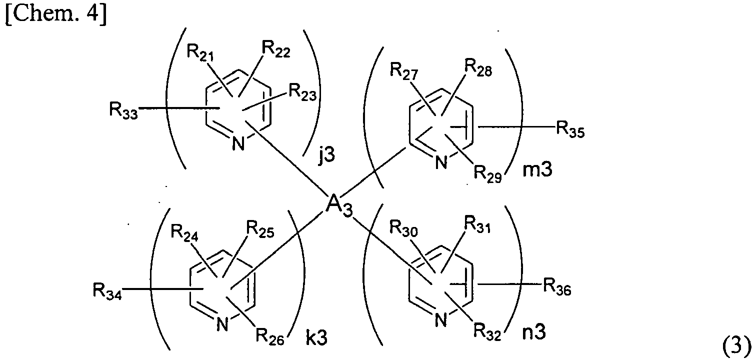

(in the formula, R₂₁ to R₃₆ may be the same or different and represent a hydrogen atom, a deuterium atom, a fluorine atom, a chlorine atom, a cyano group, a trifluoromethyl group, a linear or branched alkyl group having 1 to 6 carbon atoms which may have a substituent, a substituted or unsubstituted aromatic hydrocarbon group, a substituted or unsubstituted aromatic heterocyclic group, or a substituted or unsubstituted condensed polycyclic aromatic group; j3, k3, m3, and n3 represent an integer of 1 to 4, excluding the case where all of j3, k3, m3, and n3 have an identical value simultaneously; plurality of R₂₁ to R₃₂ present in one molecule may be the same or different from one another; and A₃ represents a tetravalent group of a substituted or unsubstituted aromatic hydrocarbon, a tetravalent group of a substituted or unsubstituted aromatic heterocycle, or a tetravalent group of a substituted or unsubstituted condensed polycyclic aromatic).

The "aromatic hydrocarbon", "aromatic heterocycle", or "condensed polycyclic aromatic" in the "substituted or unsubstituted aromatic hydrocarbon group", "substituted or unsubstituted aromatic heterocyclic group", or "substituted or unsubstituted condensed polycyclic aromatic group" represented by A₁, A₂ or A₃ in the general formula (1), (2) or (3) specifically includes benzene, biphenyl, terphenyl, tetrakisphenyl, styrene, naphthalene, anthracene, acenaphthylene, fluorene, phenanthrene, indane, pyrene, pyridine, pyrimidine, triazine, furane, pyrone, thiophene, quinoline, isoquinoline, benzofuran, benzothiophene, indoline, carbazole, benzoxazole, benzothiazole, quinoxaline, benzimidazole, pyrazole, dibenzofuran, dibenzothiophene, naphthyridine, phenanthroline, and acridine. The "di- to tetra- valent group of a substituted or unsubstituted aromatic hydrocarbon", "di- to tetravalent group of a substituted or unsubstituted aromatic heterocycle", or "di- to tetra- valent group of a substituted or unsubstituted condensed polycyclic aromatic" represented by A₁, A₂ or A₃ in the general formula (1), (2) or (3) indicates divalent, trivalent, or tetravalent group corresponding to the above "aromatic hydrocarbon", "aromatic heterocycle", or "condensed polycyclic aromatic".

The "substituent" in the "substituted aromatic hydrocarbon", "substituted aromatic heterocycle", or "substituted condensed polycyclic aromatic" represented by A₁, A₂ or A₃ in the general formula (1), (2) or (3) specifically includes a deuterium atom, a fluorine atom, a chlorine atom, a cyano group, a trifluoromethyl group, a hydroxyl group, a nitro group, a linear or branched alkyl group having 1 to 6 carbon atoms, a cyclopentyl group, a cyclohexyl group, a linear or branched alkoxy group having 1 to 6 carbon atoms, a dialkyl amino group substituted by a linear or branched alkyl group having 1 to 6 carbon atoms, a phenyl group, a naphthyl group, an anthryl group, a fluorenyl group, a styryl group, a pyridyl group, a pyridoindolyl group, a quinolyl group, and benzothiazolyl group, and these substituents may be further substituted.

The "linear or branched alkyl group having 1 to 6 carbon atoms which may have a substituent" represented by R₁ to R₃₆ in the general formula (1), (2) or (3) specifically includes a methyl group, an ethyl group, an n-propyl group, an isopropyl group, an n-butyl group, an isobutyl group, a tert-butyl group, an n-pentyl group, an isopentyl group, a neopentyl group, and an n-hexyl group. These groups may connect with each other to form a ring.

The "substituent" in the "linear or branched alkyl group having 1 to 6 carbon atoms which have a substituent" represented by R₁ to R₃₆ in the general formula (1), (2) or (3) specifically includes a deuterium atom, a fluorine atom, a chlorine atom, a cyano group, a trifluoromethyl group, a nitro group, a linear or branched alkyl group having 1 to 6 carbon atoms, a cyclopentyl group, a cyclohexyl group, a linear or branched alkoxy group having 1 to 6 carbon atoms, a dialkyl amino group substituted by a linear or branched alkyl group having 1 to 6 carbon atoms, a phenyl group, a biphenylyl group, a terphenyl group, a tetrakisphenyl group, a styryl group, a naphthyl group, a fluorenyl group, a phenanthryl group, an indenyl group, a pyrenyl group, a pyridyl group, a bipyridyl group, a triazyl group, a pyrimidyl group, a quinolyl group, an isoquinolyl group, an indolyl group, a pyridoindolyl group, a carbazolyl group, a quinoxalyl group, and a pyrazolyl group, and these substituents may be further substituted.

The "aromatic hydrocarbon group", "aromatic heterocyclic group", or "condensed polycyclic aromatic group" in the "substituted or unsubstituted aromatic hydrocarbon group", "substituted or unsubstituted aromatic heterocyclic group", or "substituted or unsubstituted condensed polycyclic aromatic group" represented by R₁ to R₃₆ in the general formula (1), (2) or (3) specifically includes a phenyl group, a biphenylyl group, a terphenylyl group, a tetrakisphenyl group, a styryl group, a naphthyl group, an anthryl group, an acenaphthenyl group, a fluorenyl group, a phenanthryl group, an indenyl group, a pyrenyl group, a pyridyl group, a bipyridyl group, a triazyl group, a pyrimidyl group, a furanyl group, a pyronyl group, a thiophenyl group, a quinolyl group, an isoquinolyl group, a benzofuranyl group, a benzothiophenyl group, an indolyl group, a carbazolyl group, a benzoxazolyl group, a benzothiazolyl group, a quinoxalyl group, a benzimidazolyl group, a pyrazolyl group, a pyridoindolyl group,a dibenzofuranyl group, a dibenzothiophenyl group, a naphthyridinyl group, a phenanthrolinyl group and an acridinyl group. These groups may connect with each other to form a ring.

The "substituent" in the "substituted aromatic hydrocarbon group", "substituted aromatic heterocyclic group", or "substituted condensed polycyclic aromatic group" represented by R₁ to R₃₆ in the general formula (1), (2) or (3) specifically includes a deuterium atom, a fluorine atom, a chlorine atom, a cyano group, a trifluoromethyl group, a nitro group, a linear or branched alkyl group having 1 to 6 carbon atoms, a cyclopentyl group, a cyclohexyl group, a linear or branched alkoxy group having 1 to 6 carbon atoms, a dialkyl amino group substituted by a linear or branched alkyl group having 1 to 6 carbon atoms, a phenyl group, a biphenylyl group, a terphenyl group, a tetrakisphenyl group, a styryl group, a naphthyl group, a fluorenyl group, a phenanthryl group, an indenyl group, a pyrenyl group, a pyridyl group, a bipyridyl group, a triazyl group, a pyrimidyl group, a quinolyl group, an isoquinolyl group, an indolyl group, a pyridoindolyl group, a carbazolyl group, a quinoxalyl group, and a pyrazolyl group, and these substituents may be further substituted.

As the combination of J1, k1, and m1 in the general formula (1) of the present invention, it is preferred that (j1, k1, m1) equals (2, 2, 3) or (3, 3, 2).

A₁ in the general formula (1) of the present invention is preferably the "trivalent group of an unsubstituted aromatic hydrocarbon", "trivalent group of an unsubstituted aromatic heterocycle", or "trivalent group represented by the above general formula (1-1)", and particularly preferably a trivalent group derived from benzene, triazine, or 2,4,6-triphenylpyridine.

The substituted pyridyl compound represented by the general formula (1), (2), or (3) of the present invention is a novel compound. Since the compound provides high electron mobility as compared with conventional electron-transporting materials, has an excellent hole-blocking ability, and has a destroyed symmetry, a thin-film state is particularly stable. The substituted pyridyl compound represented by the general formula (1), (2), or (3) of the present invention can maintain a stable thin-film state and hence provides effects of enhancing luminous efficiency and also lowering a driving voltage in the case where the compound is used as a constituent material for an organic electroluminescent element (hereinafter referred to as organic EL element).

The substituted pyridyl compound represented by the general formula (1), (2), or (3) of the present invention can be used as a constituent material for an electron-injecting layer and/or an electron-transporting layer of an organic EL element. The use of the material exhibiting a high electron injection/mobile rate as compared with conventional materials provides effects of improving electron transport efficiency from the electron-transporting layer to an emitting layer to enhance luminous efficiency and also lowering a driving voltage to enhance durability of the organic EL element.

The substituted pyridyl compound represented by the general formula (1), (2), or (3) of the present invention can be also used as a constituent material for a hole-blocking layer of an organic EL element. The use of the material excellent in hole-blocking ability and also excellent in electron transport property as compared with conventional materials and having high stability in a thin-film state provides effects of lowering a driving voltage, improving current resistance, and enhancing maximum emission luminance of the organic EL element, while exhibiting high luminous efficiency.

The substituted pyridyl compound represented by the general formula (1), (2), or (3) of the present invention can be also used as a constituent material for an emitting layer of an organic EL element. The use of an emitting layer prepared by using the material of the present invention excellent in electron transport property as compared with conventional materials and having a wide bandgap as a host material for the emitting layer and making a fluorescent material or a phosphorescent emitting material, called a dopant, carried thereon provides an effect of realizing an organic EL element exhibiting a lowered driving voltage and having improved luminous efficiency.

Since the organic EL element of the present invention uses a substituted pyridyl compound providing high electron mobility as compared with conventional electron-transporting materials, having an excellent hole-blocking ability, and being stable in a thin-film state, it becomes possible to realize a high efficiency and a high durability.

### ADVANTAGES OF THE INVENTION

Since the substituted pyridyl compound of the present invention provides high electron mobility, has an excellent hole-blocking ability, and is stable in a thin-film state, the compound is useful as a constituent material for an electron-injecting layer, an electron-transporting layer, a hole-blocking layer, or an emitting layer of an organic EL element. The organic EL element prepared by using the substituted pyridyl compound can enhance emitting efficiency and also lower a driving voltage to enhance durability.

### BRIEF DESCRIPTION OF THE DRAWINGS

[FIG. 1] FIG. 1 is a ¹H-NMR chart of the compound (Compound 13) of Invention Example 1.
[FIG. 2] FIG. 2 is a ¹H-NMR chart of the compound (Compound 37) of Invention Example 2
[FIG. 3] FIG. 3 is a drawing showing the constitution of the EL elements of Example 5, Example 6 and Comparative Example 1.

### MODE FOR CARRYING OUT THE INVENTION

The substituted pyridyl compound of the present invention is a novel compound and the compound can be synthesized, for example, by carrying out a cross-coupling reaction such as Suzuki coupling (see e.g., Non-Patent Document 7) of one of various halogenopyridines with a boronic acid or a boronate ester (see e.g., Non-Patent Document 6) which is synthesized by a reaction of a halide of one of various aromatic hydrocarbon compounds, condensed polycyclic aromatic compounds, or aromatic heterocyclic compounds with pinacolborane or bis(pinacolato)diboron. Moreover, a substituted pyridyl compound with which a triazine ring is connected can be also synthesized by performing a triazine ring-forming reaction (see e.g., Non-Patent Document 4) using sodium hydride on one of various aromatic hydrocarbon compounds, condensed polycyclic aromatic compounds, or aromatic heterocyclic compounds each having a nitrile group.

Among the substituted pyridyl compounds represented by the general formula (1), (2), or (3), specific examples of preferred compounds are shown below, but the present invention should not be construed as being limited to these compounds.

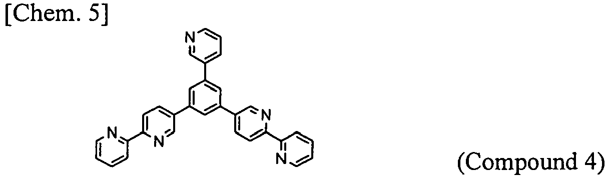

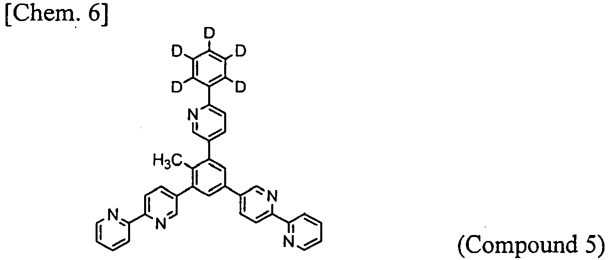

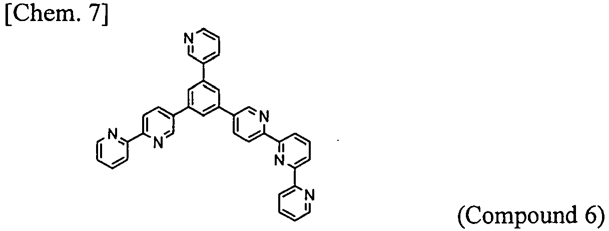

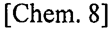

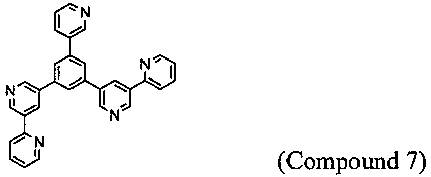

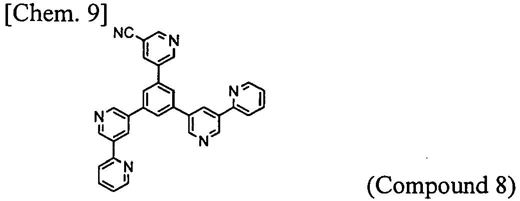

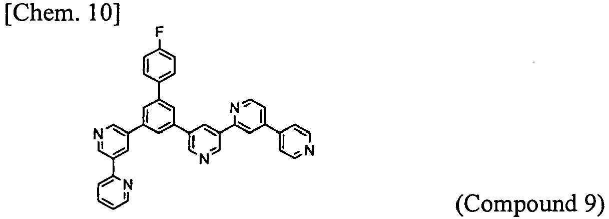

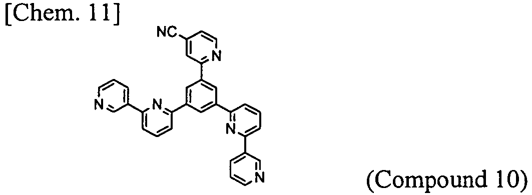

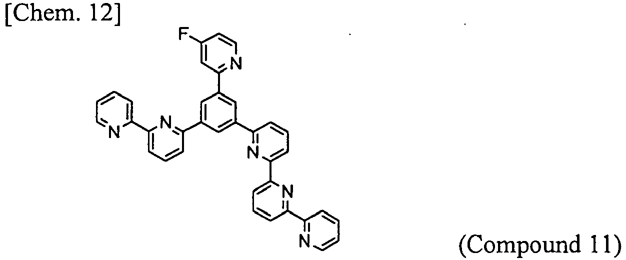

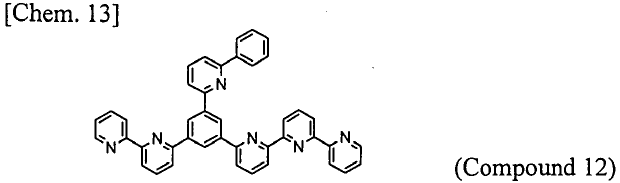

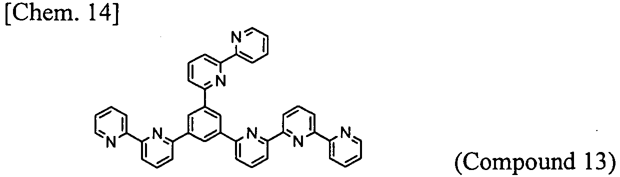

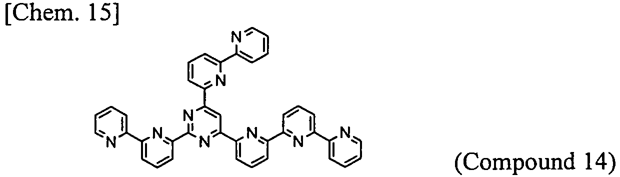

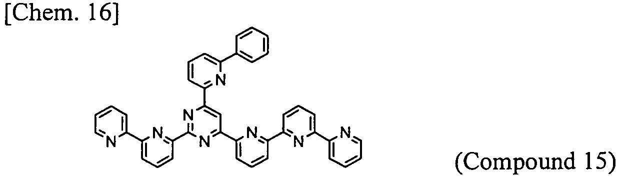

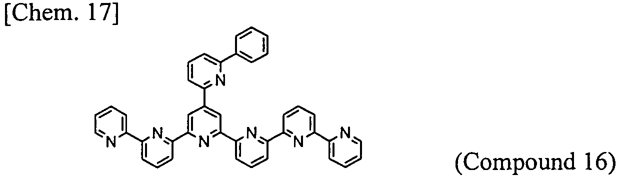

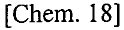

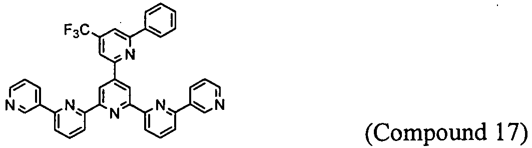

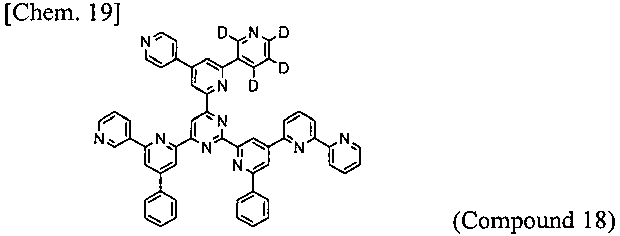

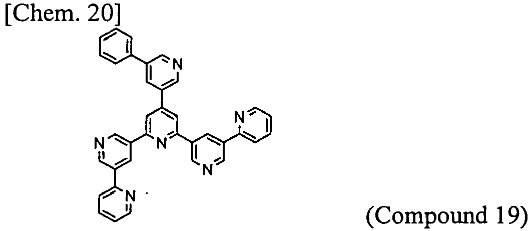

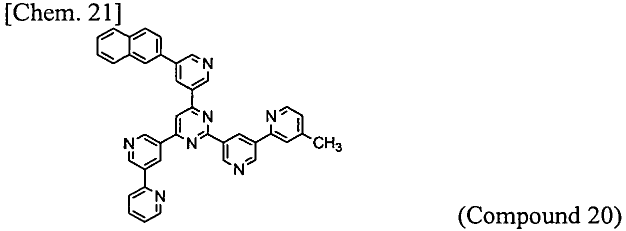

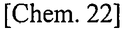

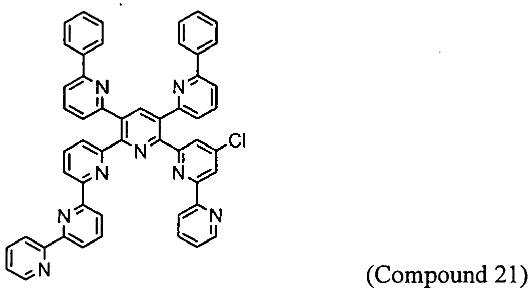

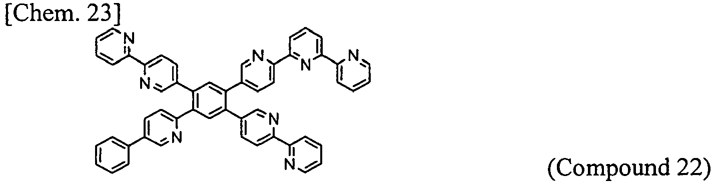

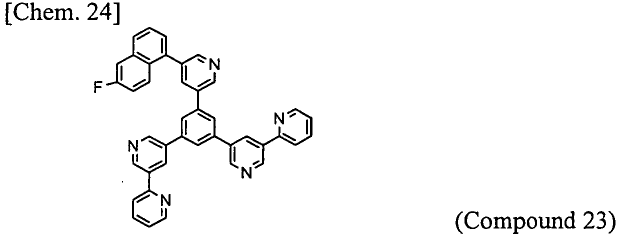

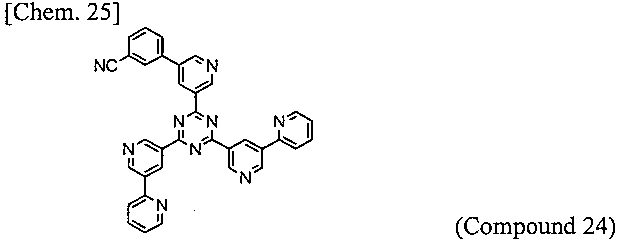

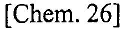

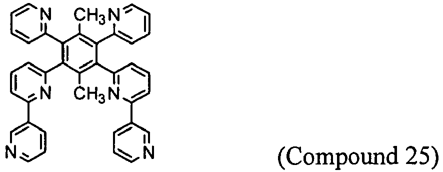

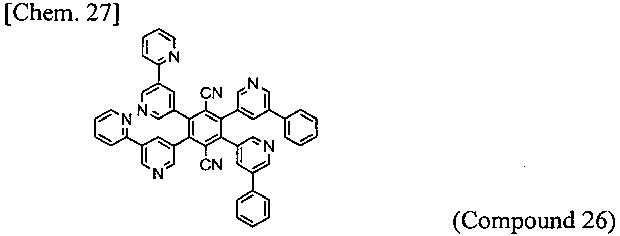

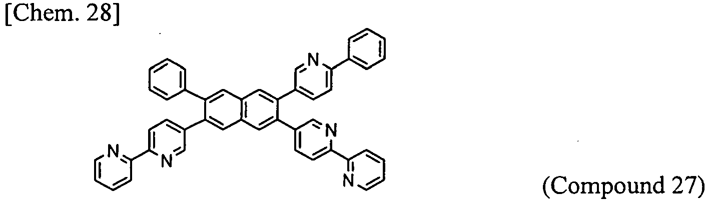

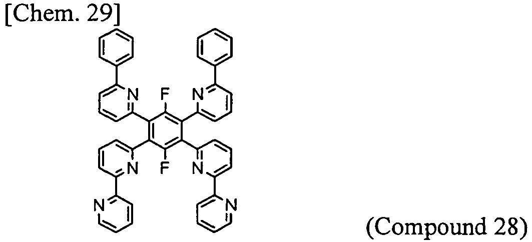

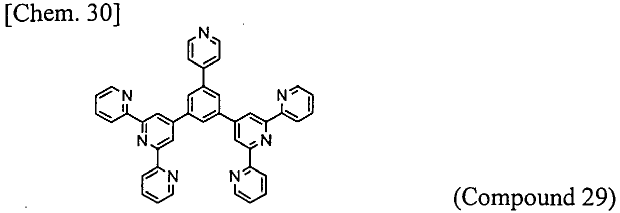

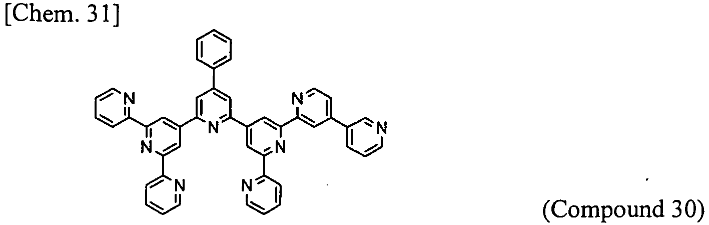

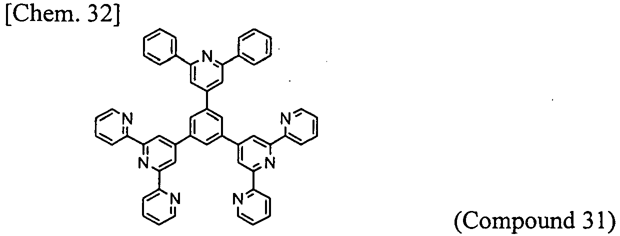

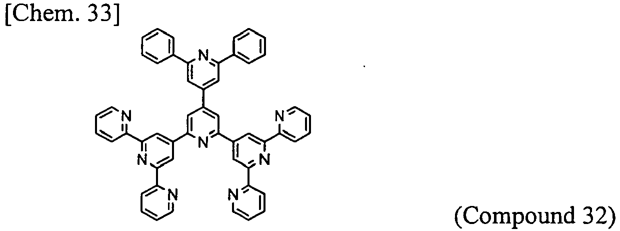

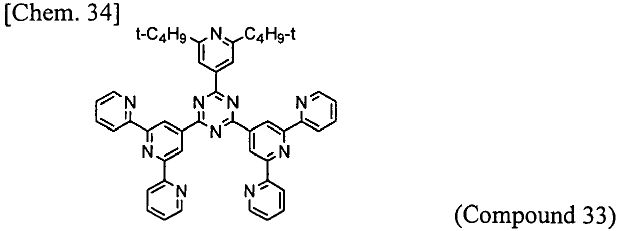

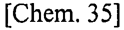

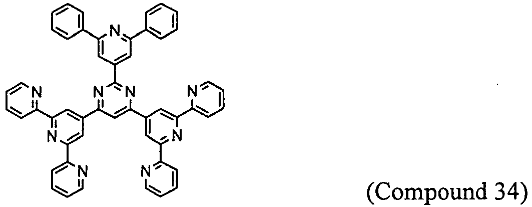

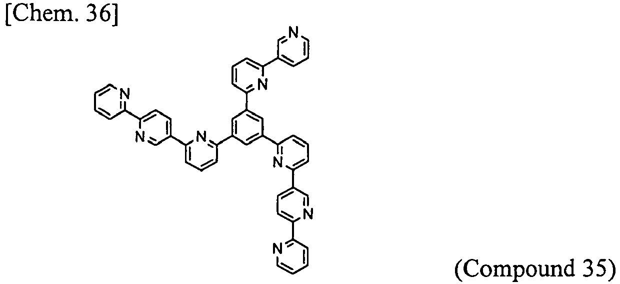

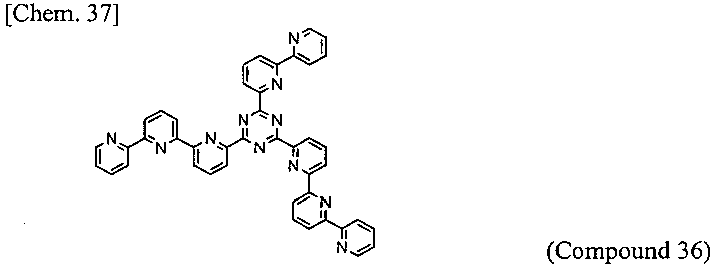

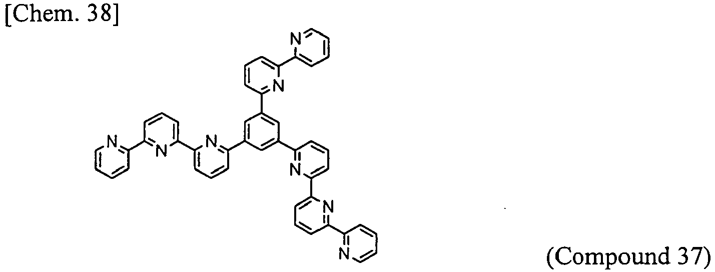

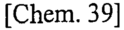

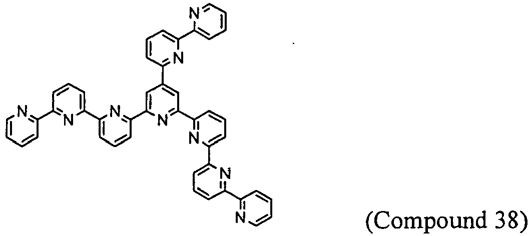

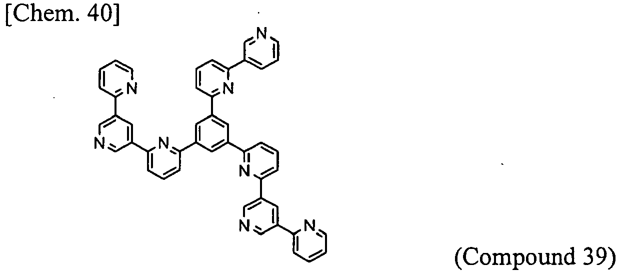

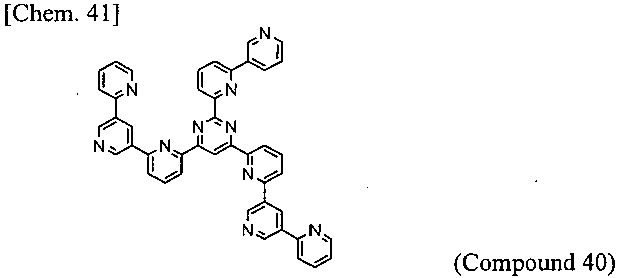

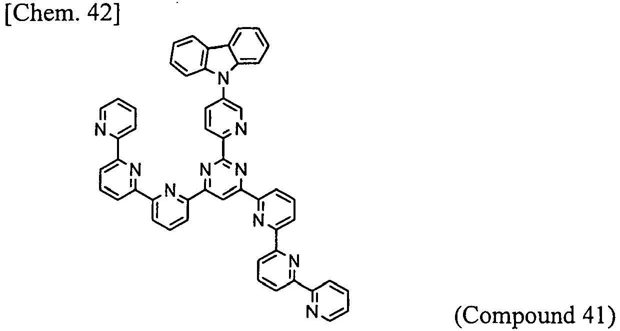

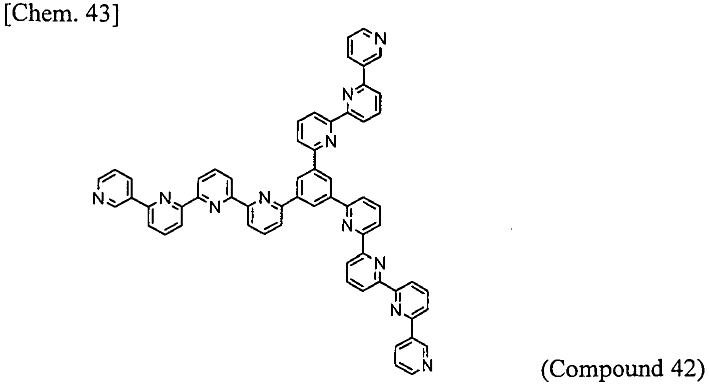

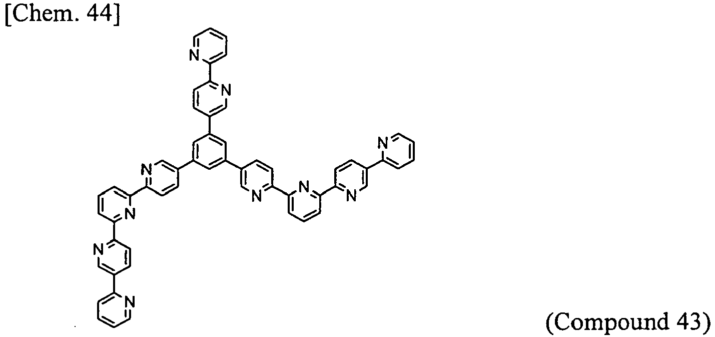

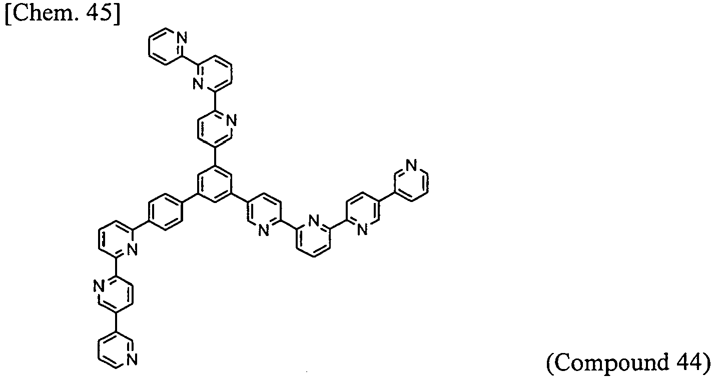

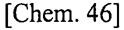

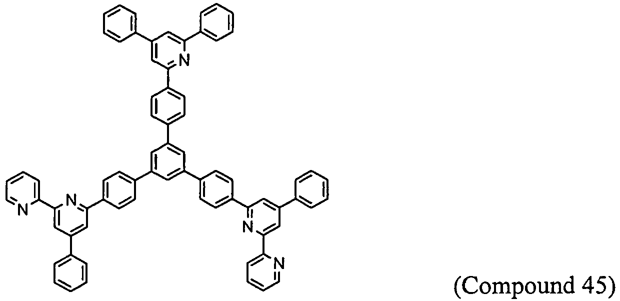

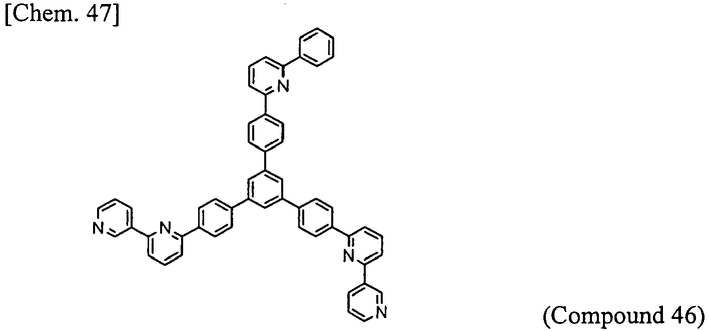

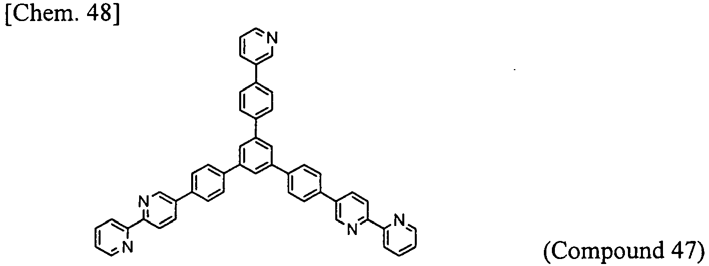

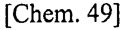

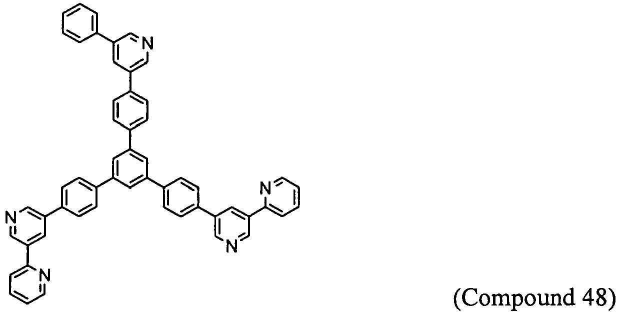

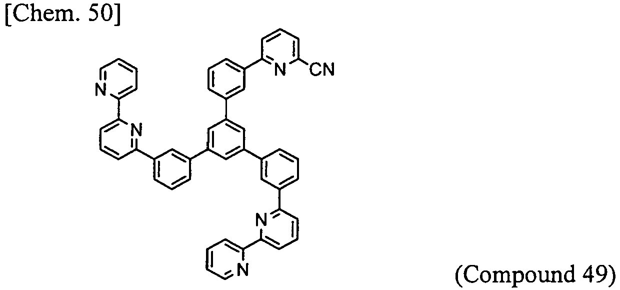

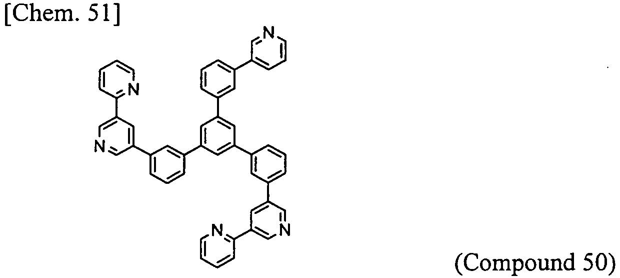

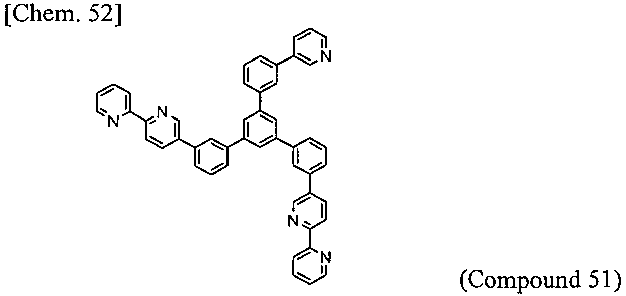

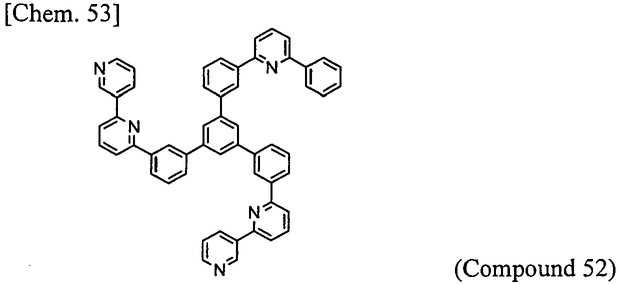

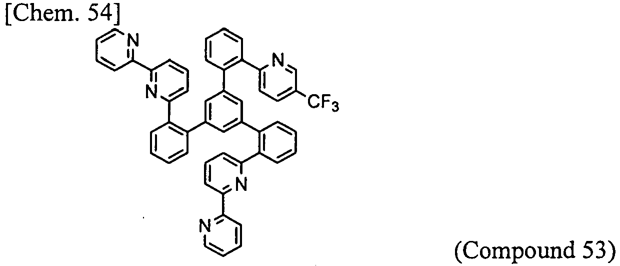

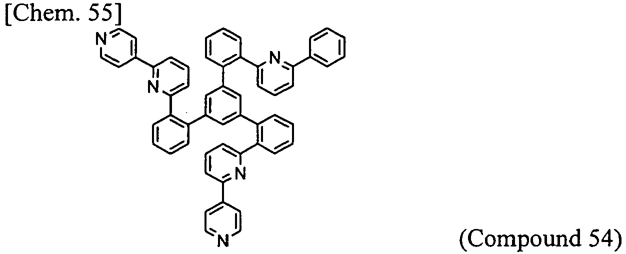

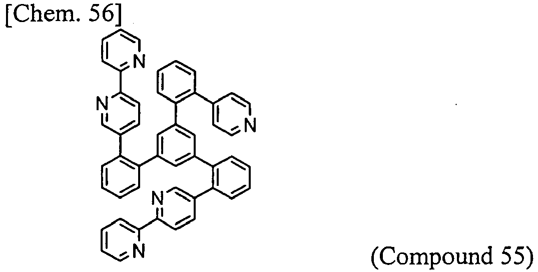

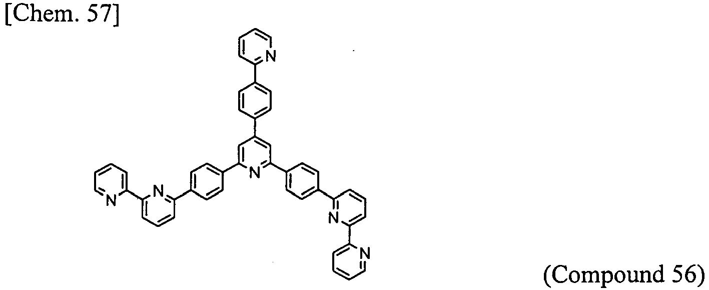

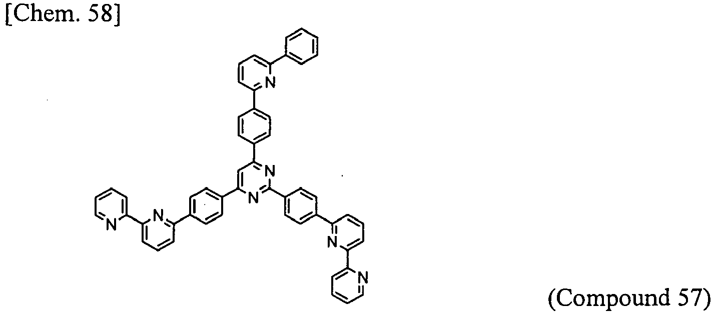

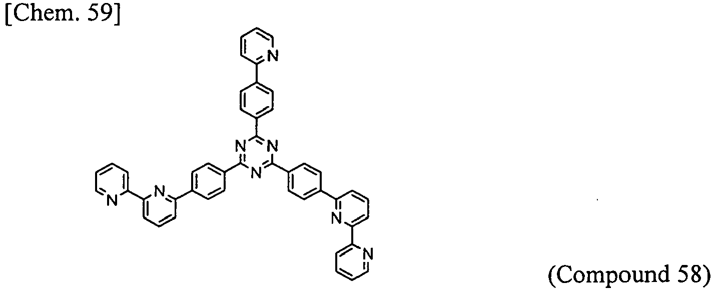

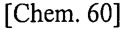

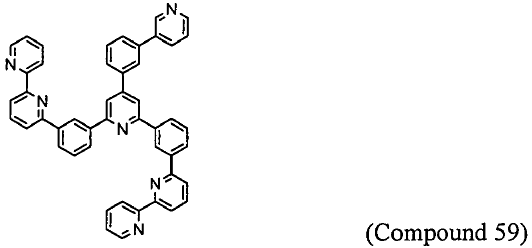

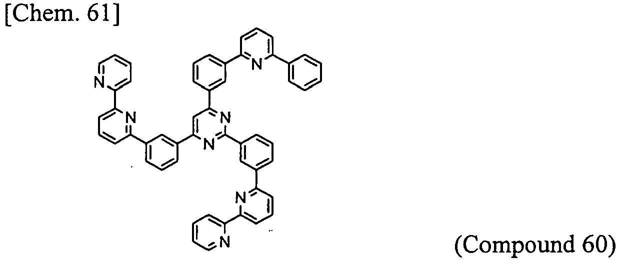

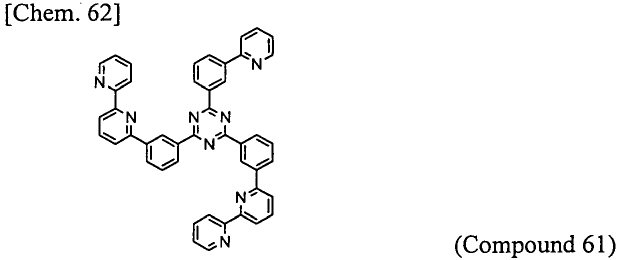

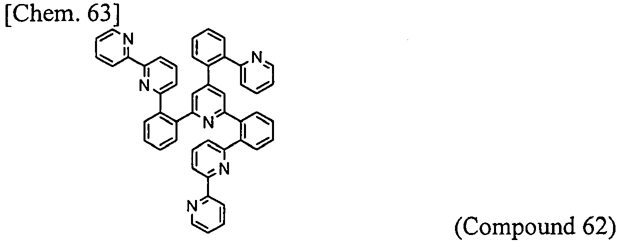

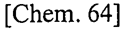

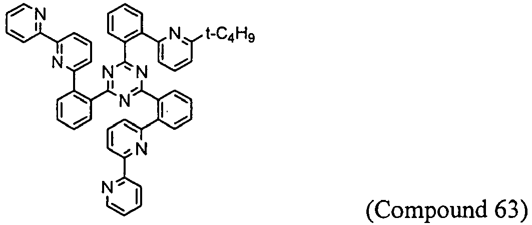

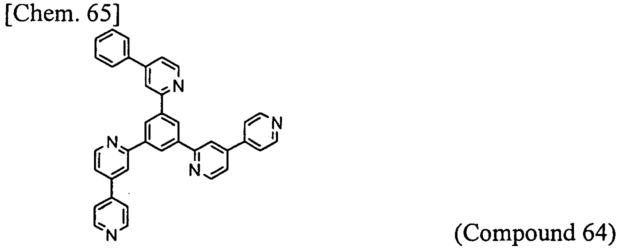

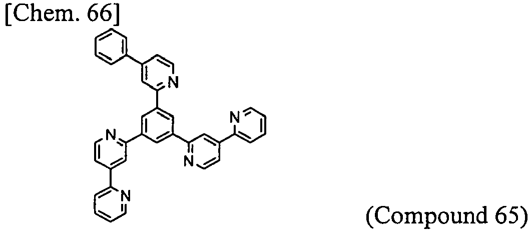

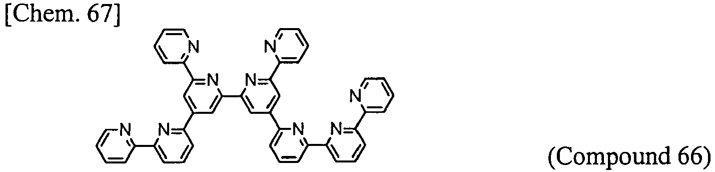

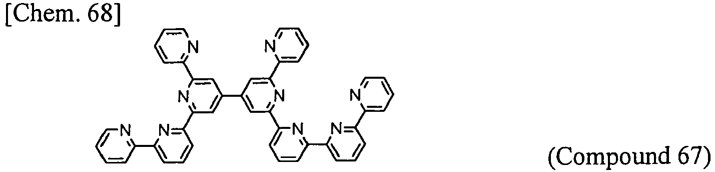

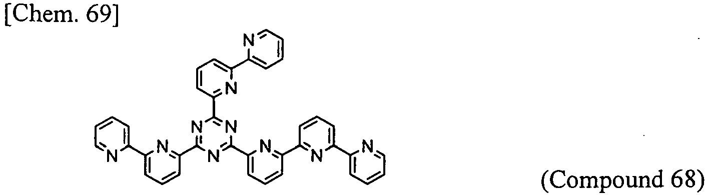

Purification of these compounds was performed by purification by column chromatography, adsorption purification with silica gel, active charcoal, activated clay or the like, a recrystallization or crystallization method with a solvent, or the like. Identification of the compounds was performed by NMR analysis. As physical properties, measurement of melting point, glass transition point (Tg), and work function was performed. The melting point serves as an index of vapor deposition properties, the glass transition point (Tg) serves as an index of stability in a thin-film state, and the work function serves as an index of hole-blocking ability.

The melting point and the glass transition point (Tg) were measured using a powder material by means of a highly sensitive differential scanning calorimeter (DSC 6200, manufactured by Seiko Instruments Inc.).

Further, the work function was measured by preparing a thin film of 100 nm on an ITO substrate and using a photo-electron spectroscopy in air (Model AC-3, manufactured by Riken Keiki Co., Ltd.).

Examples of the structure of the organic EL element of the present invention include a structure sequentially having an anode, a hole-transporting layer, an emitting layer, a hole-blocking layer, an electron-transporting layer and a cathode on a substrate, and a structure further having an hole-injecting layer between the anode and the hole-transporting layer, a structure further having an electron-injecting layer between the electron-transporting layer and the cathode, and a structure further having an electron-blocking layer between the emitting layer and the hole-transporting layer. In these multilayer structures, it is possible to omit several layers of the organic layers and, for example, the structure may have a constitution sequentially having an anode, a hole-transporting layer, an emitting layer, an electron-transporting layer and a cathode on a substrate.

In the emitting layer, hole-transporting layer, and electron-transporting layer, each layer may have a structure where two or more layers are laminated.

As the anode of the organic EL element of the present invention, an electrode material having a large work function, such as ITO or gold, is used. As the hole-injecting layer of the organic EL element of the present invention, besides porphyrin compounds including copper phthalocyanine as a representative, use can be made of star-burst type triphenylamine derivatives, triphenylamine trimers and tetramers such as arylamine compounds having in the molecule a structure in which 3 or more triphenylamine structures are connected with a single bond or a divalent group containing no hetero atom, or acceptor type heterocyclic compounds such as hexacyanoazatriphenylene, and coat-type polymer materials. These materials can be formed into a thin film by a known method such as a spin coating method or an ink jet method, in addition to a vapor deposition method.

As the hole-transporting layer of the organic EL element of the present invention, use can be made of benzidine derivatives such as N,N'-diphenyl-N,N'-di(m-tolyl)-benzidine (hereinafter referred to as TPD), N,N'-diphenyl-N,N'-di(α-naphthyl)-benzidine (hereinafter referred to as NPD), and N,N,N',N'-tetrabiphenylylbenzidine, 1,1-bis[(di-4-tolylamino)phenyl]cyclohexane (hereinafter referred to as TAPC), various triphenylamine trimers and tetramers, or the like. Each of them may be singly formed into a film but may be mixed with another material to use as a film-formed single layer or may be formed as a laminated structure of singly film-formed layers, of mixed and film-formed layers, or of a singly film-formed layer and a mixed and film-formed layer. Further, as the hole-injecting/transporting layers, use can be made of coat-type polymer materials such as poly(3,4-ethylenedioxythiophene) (hereinafter referred to as PEDOT)/poly(styrenesulfonate) (hereinafter referred to as PSS). These materials can be formed into a thin film by a known method such as a spin coating method or an ink jet method, in addition to a vapor deposition method.

Moreover, in the hole-injecting layer or hole-transporting layer, use can be made of materials obtained by further P doping oftrisbromophenylamine hexachloroantimony to the materials usually used for the layers, polymer compounds having a TPD structure as a partial structure thereof, and the like.

As the electron-blocking layer of the organic EL layer of the present invention, use can be made of compounds having an electron-blocking action such as carbazole derivatives such as 4,4',4"-tri(N-carbazolyl)triphenylamine (hereinafter referred to as TCTA), 9,9-bis[4-(carbazol-9-yl)phenyl]fluorene, 1,3-bis(carbazol-9-yl)benzene (hereinafter referred to as mCP), 2,2-bis(4-carbazole-9-ylphenyl)adamantane (hereinafter referred to as Ad-Cz) or compounds having a triphenylsilyl group and a triarylamine structure including 9-[4-(carbazol-9-yl)phenyl]-9-[4-(triphenylsilyl)phenyl]-9H-fluorene as a representative. Each of them may be singly formed into a film but may be mixed with another material to use as a film-formed single layer or may be formed as a laminated structure of singly film-formed layers, of mixed and film-formed layers, or of a singly film-formed layer and a mixed and film-formed layer. These materials can be formed into a thin film by a known method such as a spin coating method or an ink jet method, in addition to a vapor deposition method.

As the emitting layer of the organic EL element of the present invention, besides the substituted pyridyl compounds of the present invention, use can be made of various metal complexes in addition to metal complexes of quinolinol derivatives including Alq₃, anthracene derivatives, bisstyrylbenzene derivatives, pyrene derivatives, oxazole derivatives, poly-p-phenylenevinylene derivatives, or the like. Further, the emitting layer may be formed of a host material and a dopant material. As the host material, in addition to the above emitting materials, use can be made of thiazole derivatives, benzimidazole derivatives, polydialkylfluorene derivatives, or the like. Moreover, as the dopant material, use can be made of quinacridone, coumarin, rubrene, perylene, and derivatives thereof, benzopyran derivatives, rhodamine derivatives, aminostyryl derivatives, or the like. Each of them may be singly formed into a film but may be mixed with another material to use as a film-formed single layer or may be formed as a laminated structure of singly film-formed layers, of mixed and film-formed layers, or of a singly film-formed layer and a mixed and film-formed layer.

Moreover, it is also possible to use a phosphorescent emitting material as the emitting material. As the phosphorescent emitting material, use can be made of phosphorescent emitting materials of complexes of metals such as iridium and platinum. Green phosphorescent emitting materials such as Ir(ppy)₃, blue phosphorescent emitting materials such as FIrpic and FIr6, red phosphorescent emitting materials such as Btp₂Ir(acac), and the like are used. As the host material on this occasion, as hole-injecting/transporting host materials, use can be made of carbazole derivatives such as 4,4'-di(N-carbazolyl)biphenyl (hereinafter referred to as CBP), TCTA, and mCP. As an electron-transporting host material, use can be made of p-bis(triphenylsilyl)benzene (hereinafter referred to as UGH2), 2,2',2"-(1,3,5-phenylene)-tris(1-phenyl-1H-benzimidazole) (hereinafter referred to as TPBI), and the like.

At the doping of the phosphorescent emitting material to the host material, in order to avoid concentration quenching, it is preferred to perform the doping by co-deposition in the range of 1 to 30% by weight based on the whole emitting layer.

These materials can be formed into a thin film by a known method such as a spin coating method or an ink jet method, in addition to a vapor deposition method.

As the hole-blocking layer of the organic EL element of the present invention, besides the substituted pyridyl compounds of the present invention, use can be made of compounds having a hole-blocking action, such as various rare-earth complexes, oxazole derivatives, triazole derivatives, or triazine derivatives, in addition to phenanthroline derivatives such as bathocuproine (hereinafter referred to as BCP) and metal complexes of quinolinol derivatives such as BAlq. These materials may be simultaneously materials for the electron-transporting layer. Each of them may be singly formed into a film but may be mixed with another material to use as a film-formed single layer or may be formed as a laminated structure of singly film-formed layers, of mixed and film-formed layers, or of a singly film-formed layer and a mixed and film-formed layer. These materials can be formed into a thin film by a known method such as a spin coating method or an ink jet method in addition to a vapor deposition method.

As the electron-transporting layer of the organic EL element of the present invention, besides the substituted pyridyl compounds of the present invention, use can be made of various metal complexes, triazole derivatives, triazine derivatives, oxadiazole derivatives, thiadiazole derivatives, carbodiimide derivatives, quinoxaline derivatives, phenanthroline derivatives, silole derivatives, or the like, in addition to metal complexes of qunolinol derivatives including Alq₃ and BAlq. Each of them may be singly formed into a film but may be mixed with another material to use as a film-formed single layer or may be formed as a laminated structure of singly film-formed layers, of mixed and film-formed layers, or of a singly film-formed layer and a mixed and film-formed layer. These materials can be formed into a thin film by a known method such as a spin coating method or an ink jet method, in addition to a vapor deposition method.

As the electron-injecting layer of the organic EL element of the present invention, besides the substituted pyridyl compounds of the present invention, use can be made of alkali metal salts such as lithium fluoride and cesium fluoride, alkaline earth metal salts such as magnesium fluoride, metal oxides such as aluminum oxide, or the like. However, in preferable selection of the electron-transporting layer and the cathode, the layer can be omitted.

Furthermore, in the electron-injecting layer or the electron-transporting layer, use can be made of materials obtained by further N doping of a metal such as cesium to the materials usually used for the layers.

As the cathode of the organic EL element of the present invention, an electrode material having a low work function such as aluminum, or an alloy having a further low work function such as magnesium indium alloy or aluminum magnesium alloy is used as an electrode material.

Embodiments of the present invention will be illustrated below in greater detail with reference to Examples, but the present invention should not be construed as being limited to the following Examples.

### Example 1

### <Synthesis of 1,3-bis(2,2'-bipyridin-6-yl)-5-(2,2';6',2"-terpyridin-6-yl)benzene (Compound 13)>

To a reaction vessel subjected to replacement with nitrogen were added 4.5 g of 3,5-dibromophenylboronic acid, 5.0 g of 6-bromo-[2,2';6',2"]-terpyridine, 24.1 ml of a 2M aqueous potassium carbonate solution, 0.9 g of tetrakis(triphenylphosphine)palladium(0), 200 ml of toluene, and 50 ml of ethanol, and the whole was heated and refluxed under stirring for 10 hours. After cooling to room temperature, 100 ml of water and 100 ml of toluene were added thereto, the resulting liquid was separated, and the resulting organic layer was further washed with 100 ml of water. After the organic layer was subjected to water removal over anhydrous magnesium sulfate, a crude product was obtained by concentration. The crude product was purified by column chromatography (carrier: NH silica gel, eluent: toluene/n-hexane) to obtain 5.7 g (yield 75%) of 1,3-dibromo-5-(2,2';6',2"-terpyridin-6-yl)benzene as a white powder.

To a reaction vessel subjected to replacement with nitrogen were added 8.0 g of the obtained 1,3-dibromo-5-(2,2';6',2"-terpyridin-6-yl)benzene, 10.4 g of bis(pinacolato)diboron, 10.1 g of potassium acetate, 240 ml of 1,4-dioxane which had been subjected to water removal over molecular sieves 4A beforehand, and 0.9 g of PdCl₂(dppf)-CH₂Cl₂, and the whole was heated, followed by stirring at 80°C for 9 hours. After cooling to room temperature, the reaction solution was added to 1500 ml of water and the whole was stirred for 30 minutes. The resulting precipitate was collected by filtration and the precipitate was washed with methanol to obtain a crude product. After the crude product was dissolved in 200 ml of toluene, insoluble matter was removed by filtration, the resulting filtrate was concentrated, and crystallization with hexane was performed, thereby obtaining 7.8 g (yield 81%) of 1,3-bis(4,4,5,5-tetramethyl-[1,3,2]dioxaborolan-2-yl)-5-(2,2';6',2"-terpyridin-6-yl)benzene as a yellow-white powder.

To a reaction vessel subjected to replacement with nitrogen were added 7.5 g of the obtained 1,3-bis(4,4,5,5-tetramethyl-[1,3,2]dioxaborolan-2-yl)-5-(2,2';6',2"-terpyridin-6-yl)benzene, 7.0 g of 6-bromo-[2,2']-bipyridine, 20.1 ml of a 2M aqueous potassium carbonate solution, 0.8 g of tetrakis(triphenylphosphine)palladium(0), 120 ml of toluene, and 30 ml of ethanol, and the whole was heated and refluxed under stirring for 24 hours. After cooling to room temperature, 100 ml of water and 100 ml of toluene were added thereto, the resulting liquid was separated, and the resulting organic layer was further washed with 100 ml of water. After the organic layer was subjected to water removal over anhydrous magnesium sulfate, a crude product was obtained by concentration. The crude product was purified by column chromatography (carrier: NH silica gel, eluent: chloroform/n-hexane) to obtain 5.9 g (yield 71%) of 1,3-bis(2,2'-bipyridin-6-yl)-5-(2,2';6',2"-terpyridin-6-yl)benzene (Compound 13) as a white powder.

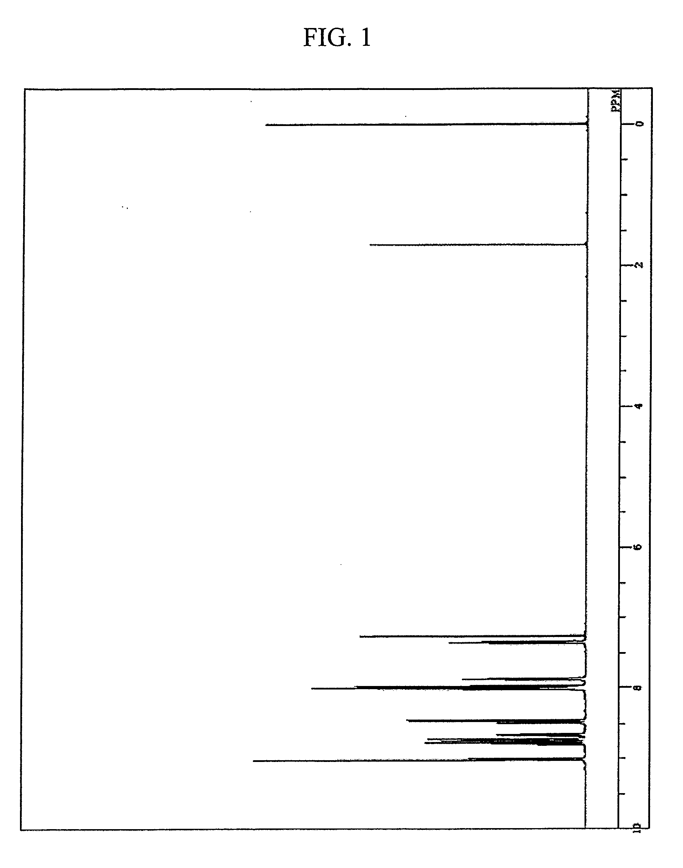

The structure of the resulting white powder was identified using NMR. The results of ¹H-NMR measurement are shown in FIG. 1.

The following 27 hydrogen signals were detected on ¹H-NMR (CDCl₃). δ(ppm) = 9.03 (2H), 9.00 (1H), 8.79 (1H), 8.76 (2H), 8.73 (3H), 8.66 (2H), 8.49 (1H), 8.46 (2H), 7.96-8.03 (7H), 7.88 (3H), 7.35 (3H).

### Example 2

### <Synthesis of 1,3-bis(2,2';6',2"-terpyridin-6-yl)-5-(2,2'-bipyridin-6-yl)benzene (Compound 37)>

By performing the same operations as in Example 1, 1,3-bis(4,4,5,5-tetramethyl-[1,3,2]dioxaborolan-2-yl)-5-(2,2'-bipyridin-6-yl)benzene was synthesized from 3,5-dibromophenylboronic acid and 6-bromo-2,2'-bipyridine. To a reaction vessel subjected to replacement with nitrogen were added 5.0 g of the obtained 1,3-bis(4,4,5,5-tetramethyl-[1,3,2]dioxaborolan-2-yl)-5-(2,2'-bipyridin-6-yl)benzene, 7.1 g of 6-bromo-[2,2';6',2"]-terpyridine, 15.5 ml of a 2M aqueous potassium carbonate solution, 0.6 g of tetrakis(triphenylphosphine)palladium(0), 40 ml of toluene, and 10 ml of ethanol, and the whole was heated and refluxed under stirring for 27 hours. After cooling to room temperature, 100 ml of water and 100 ml of toluene were added thereto, the resulting liquid was separated, and the resulting organic layer was further washed with 100 ml of water. After the organic layer was subjected to water removal over anhydrous magnesium sulfate, a crude product was obtained by concentration. The crude product was purified by column chromatography (carrier: NH silica gel, eluent: toluene) to obtain 2.0 g (yield 28%) of 1,3-bis(2,2';6',2"-terpyridin-6-yl)-5-(2,2'-bipyridin-6-yl)benzene (Compound 37) as a white powder.

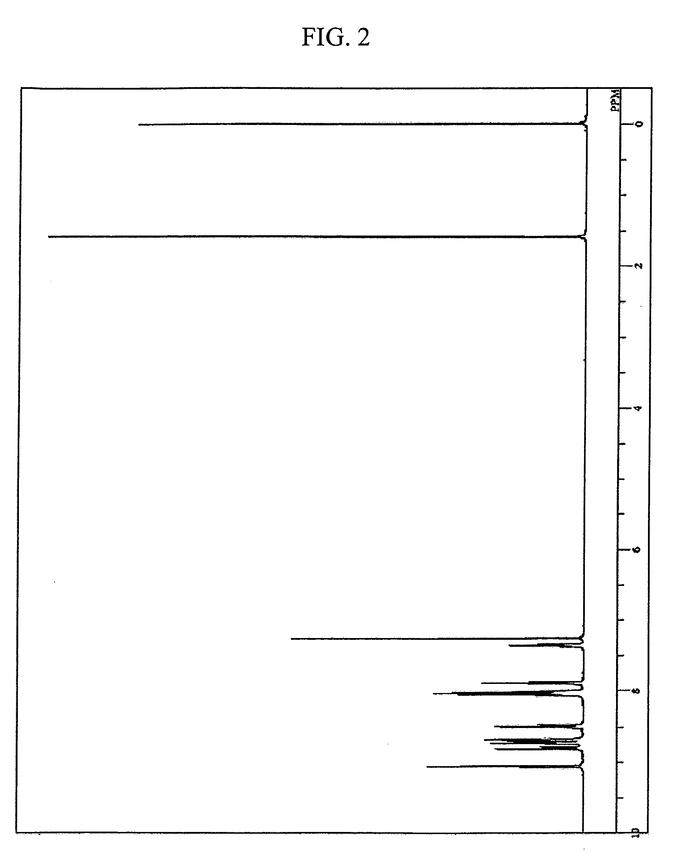

The structure of the resulting white powder was identified using NMR. The results of ¹H-NMR measurement are shown in FIG. 2.

The following 30 hydrogen signals were detected on ¹H-NMR (CDCl₃). δ (ppm) = 9.07 (1H), 9.05 (2H), 8.82 (2H), 8.79 (1H), 8.74 (3H), 8.69 (4H), 8.51 (2H), 8.48 (1H), 7.99-8.06 (8H), 7.90 (3H), 7.36 (3H).

### Example 3

For the compounds of the present invention, melting point and glass transition point were determined by means of a highly sensitive differential scanning calorimeter (DSC 6200, manufactured by Seiko Instruments Inc.).

| | Melting Point | Glass Transition Point |
|---|---|---|
| Compound of Invention Example 1 | 226°C | 96°C |
| Compound of Invention Example 2 | 235°C | 108°C |

The compounds of the present invention show a glass transition point of 80°C or higher. This fact shows that the compounds of the present invention are stable in a thin-film state. Further, the compounds of the present invention have a melting point of 200°C or higher. This fact shows that the compounds of the present invention are excellent in vapor deposition property.

### Example 4

Using each of the compounds of the present invention, a deposited film having a film thickness of 100 nm was prepared on an ITO substrate and work function was measured on a photo-electron spectroscopy in air (Model AC-3, manufactured by Riken Keiki Co., Ltd.).

| | Work function |
|---|---|
| Compound of Invention Example 1 | 6.33 eV |
| Compound of Invention Example 2 | 6.50 eV |

Thus, the compounds of the present invention have values deeper than a work function of 5.4 eV possessed by common hole-transporting materials such as NPD and TPD and have a large hole-blocking ability.

### Example 5

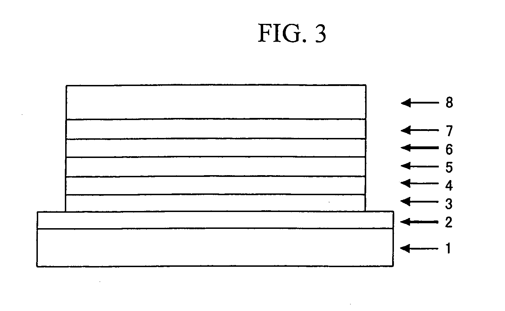

An organic EL element was prepared by depositing a hole-transporting layer 3, an emitting layer 4, a hole-blocking layer 5, an electron-transporting layer 6, an electron-injecting layer 7, and a cathode (aluminum electrode) 8 in this order on a glass substrate 1 on which an ITO electrode had been formed as a transparent anode 2 in advance, as shown in FIG. 3.

Specifically, after the glass substrate 1 on which ITO having a film thickness of 150 nm had been formed was washed with an organic solvent, the surface thereof was cleaned by UV ozone treatment. Then, the ITO electrode-fitted glass substrate was mounted in a vacuum deposition machine, which was then evacuated to 0.001 Pa or lower. Subsequently, NPD was formed thereon at a deposition rate of 6 nm/min to a thickness of 50 nm as the hole-transporting layer 3 so as to cover the transparent anode 2. As the emitting layer 4, Alq₃ was formed on the hole-transporting layer 3 at a deposition rate of 6 nm/min so as to be a thickness of 30 nm. On the emitting layer 4, the compound of Invention Example 1 (Compound 13) was formed at a deposition rate of 6 nm/min so as to be a thickness of 20 nm as the hole-blocking layer 5-cum-electron-transporting layer 6. On the hole-blocking layer 5-cum-electron-transporting layer 6, lithium fluoride was formed at a deposition rate of 0.6 nm/min so as to be a thickness of 0.5 nm as the electron-injecting layer 7. Finally, aluminum was deposited so as to be a thickness of 200 nm to form the cathode 8. The thus prepared organic EL element was stored in a vacuum desiccator and characteristic properties were measured in the atmosphere at ordinary temperature.

The results of measuring luminescence properties when a current having a current density of 10 mA/cm² was applied to the organic EL element prepared using the compound of Example 1 (Compound 13) of the present invention are summarized in Table 1.

### Example 6

An organic EL element was prepared under the same conditions as in Example 5 except that the material of the hole-blocking layer 5-cum-electron-transporting layer 6 was replaced by the compound of Invention Example 2 (Compound 37) in Example 5. For the prepared organic EL element, characteristic properties were measured in the atmosphere at ordinary temperature.
The results of measuring luminescence properties when a current having a current density of 10 mA/cm² was applied to the prepared organic EL element are summarized in Table 1.

### [Comparative Example 1]

For comparison, an organic EL element was prepared in the same manner as in Example 5 except that the material of the hole-blocking layer 5-cum-electron-transporting layer 6 in Example 5 was replaced by Alq₃ as the electron-transporting layer 6. The results of measuring luminescence properties when a current having a current density of 10 mA/cm² was applied to the prepared organic EL element are summarized in Table 1.

**[Table 1]**

| | | Driving voltage [V] (@10mA/cm²) | Luminance [cd/m²] (@10mA/cm²) | Luminous efficiency [cd/A] (@10mA/cm²) | Power efficiency [lm/W] (@10mA/cm²) |
|---|---|---|---|---|---|
| Example 5 | Compound 13 | 3.91 | 303 | 3.03 | 2.43 |
| Example 6 | Compound 37 | 3.80 | 294 | 2.94 | 2.41 |
| Comparative Example 1 | Alq₃ | 4.95 | 297 | 2.97 | 1.88 |

As shown in Table 1, the driving voltage when a current having a current density of 10 mA/cm² was applied was decreased to a low voltage of 3.91 V in the case where the compound of Invention Example 1 (Compound 13) was used as the hole-blocking layer 5-cum-electron-transporting layer 6 and 3.80 V in the case where the compound of Invention Example 2 (Compound 37) was used as the hole-blocking layer 5-cum-electron-transporting layer 6 as compared to 4.95 V in the case where Alq₃ was used as the electron-transporting layer. Moreover, luminance and luminous efficiency when a current having a current density of 10 mA/cm² was applied showed almost equal values. Power efficiency was remarkably enhanced to 2.43 lm/W in the case where the compound of Invention Example 1 (Compound 13) was used as the hole-blocking layer 5-cum-electron-transporting layer 6 and 2.41 lm/W in the case where the compound of invention Example 2 (Compound 37) was used as the hole-blocking layer 5-cum-electron-transporting layer 6 as compared to 1.88 lm/W in the case where Alq₃ was used as the electron-transporting layer.

As is apparent from these results, it was revealed that the organic EL elements using the substituted pyridyl compounds of the present invention can achieve large enhancement of powder efficiency and remarkable decrease in practical driving voltage, as compared with the elements using Alq₃ which is a commonly employed general electron-transporting material.

From the remarkable decrease in driving voltage in the organic EL element using the substituted pyridyl compound of the present invention as above, it is estimated that the electron transfer rate of the substituted pyridyl compound of the present invention is dramatically high as compared with Alq₃ which is a general electron-transporting material.

While the present invention has been described in detail and with reference to specific embodiments thereof, it will be apparent to one skilled in the art that various changes and modifications can be made therein without departing from the spirit and scope thereof.
The present application is based on Japanese Patent Application No. 2010-039565 filed on February 25, 2010, and the contents thereof are incorporated herein by reference.

### INDUSTRIAL APPLICABILITY

Since the substituted pyridyl compound of the present invention exhibits a good injection and transportation performance of electrons and also an excellent hole-blocking ability and is stable in a thin-film state, the compound is excellent as a compound for organic EL elements. By preparing organic EL elements using the compound, a high efficiency can be obtained as well as practical driving voltage can be decreased and durability can be improved. For example, it becomes possible to spread the applications onto electric home appliances and illumination.

### DESCRIPTION OF REFERENCE NUMERALS AND SIGNS

1: Glass substrate
2: Transparent anode
3: Hole-transporting layer
4: Emitting layer
5: Hole-blocking layer
6: Electron-transporting layer
7: Electron-injecting layer
8: Cathode

## Claims

1. A substituted pyridyl compound represented by the following general formula (1), (2), or (3): 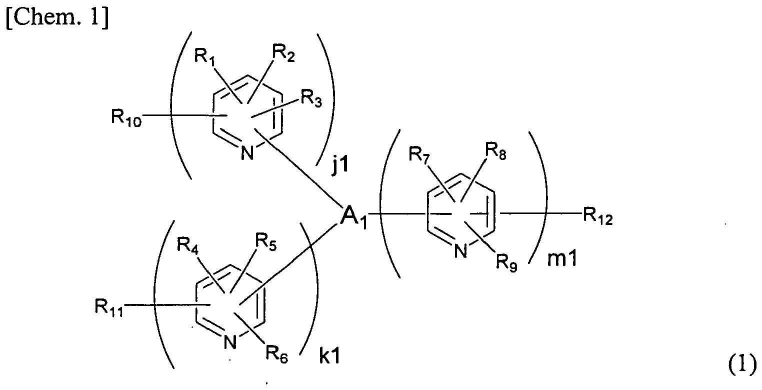 (wherein R₁ to R₁₂ may be the same or different and represent a hydrogen atom, a deuterium atom, a fluorine atom, a chlorine atom, a cyano group, a trifluoromethyl group, a linear or branched alkyl group having 1 to 6 carbon atoms which may have a substituent, a substituted or unsubstituted aromatic hydrocarbon group, a substituted or unsubstituted aromatic heterocyclic group, or a substituted or unsubstituted condensed polycyclic aromatic group; j 1, k1, and m1 represent an integer of 1 to 4, excluding the case where all of j1, k1, and m1 have an identical value simultaneously; plurality of R₁ to R₉ present in one molecule may be the same or different from one another; and A₁ represents a trivalent group of a substituted or unsubstituted aromatic hydrocarbon, a trivalent group of a substituted or unsubstituted aromatic heterocycle, a trivalent group of a substituted or unsubstituted condensed polycyclic aromatic, or a trivalent group represented by the following general formula (1-1): 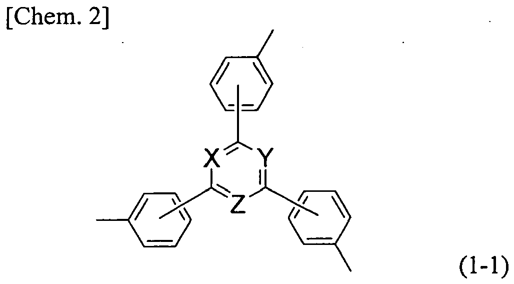 (wherein X, Y, and Z represent a carbon atom or a nitrogen atom)); 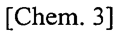 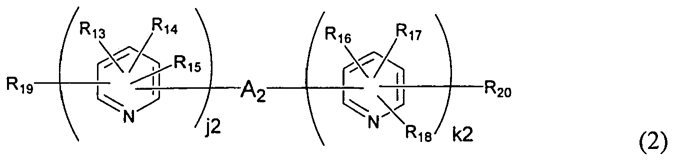 (wherein R₁₃ to R₂₀ may be the same or different and represent a hydrogen atom, a deuterium atom, a fluorine atom, a chlorine atom, a cyano group, a trifluoromethyl group, a linear or branched alkyl group having 1 to 6 carbon atoms which may have a substituent, a substituted or unsubstituted aromatic hydrocarbon group, a substituted or unsubstituted aromatic heterocyclic group, or a substituted or unsubstituted condensed polycyclic aromatic group; j2 and k2 represent an integer of 3 to 5, excluding the case where j2 and k2 have an identical value simultaneously; plurality of R₁₃ to R₁₈ present in one molecule may be the same or different from one another; and A₂ represents a divalent group of a substituted or unsubstituted aromatic hydrocarbon, a divalent group of a substituted or unsubstituted aromatic heterocycle, a divalent group of a substituted or unsubstituted condensed polycyclic aromatic, or a single bond); and 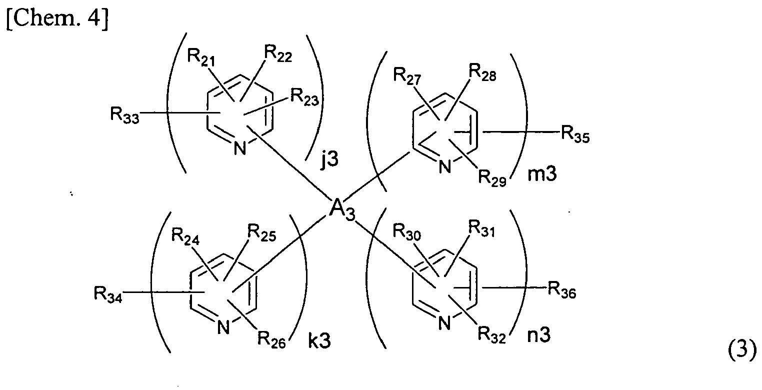 (wherein R₂₁ to R₃₆ may be the same or different and represent a hydrogen atom, a deuterium atom, a fluorine atom, a chlorine atom, a cyano group, a trifluoromethyl group, a linear or branched alkyl group having 1 to 6 carbon atoms which may have a substituent, a substituted or unsubstituted aromatic hydrocarbon group, a substituted or unsubstituted aromatic heterocyclic group, or a substituted or unsubstituted condensed polycyclic aromatic group; j3, k3, m3, and n3 represent an integer of 1 to 4, excluding the case where all of j 3, k3, m3, and n3 have an identical value simultaneously; plurality of R₂₁ to R₃₂ present in one molecule may be the same or different from one another; and A₃ represents a tetravalent group of a substituted or unsubstituted aromatic hydrocarbon, a tetravalent group of a substituted or unsubstituted aromatic heterocycle, or a tetravalent group of a substituted or unsubstituted condensed polycyclic aromatic).

2. The substituted pyridyl compound according to claim 1, represented by the following general formula (1-2): 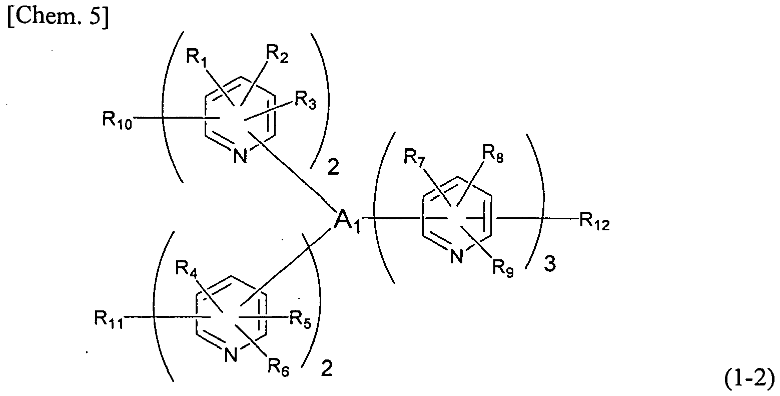 (wherein R₁ to R₁₂ may be the same or different and represent a hydrogen atom, a deuterium atom, a fluorine atom, a chlorine atom, a cyano group, a trifluoromethyl group, a linear or branched alkyl group having 1 to 6 carbon atoms which may have a substituent, a substituted or unsubstituted aromatic hydrocarbon group, a substituted or unsubstituted aromatic heterocyclic group, or a substituted or unsubstituted condensed polycyclic aromatic group; plurality of R₁ to R₉ present in one molecule may be the same or different from one another; and A₁ represents a trivalent group of a substituted or unsubstituted aromatic hydrocarbon, a trivalent group of a substituted or unsubstituted aromatic heterocycle, or a trivalent group of a substituted or unsubstituted condensed polycyclic aromatic).

3. The substituted pyridyl compound according to claim 1, represented by the following general formula (1-3): 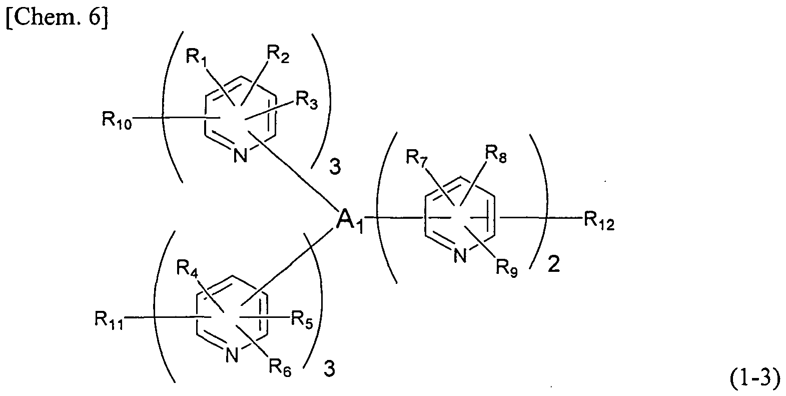 (wherein R₁ to R₁₂ may be the same or different and represent a hydrogen atom, a deuterium atom, a fluorine atom, a chlorine atom, a cyano group, a trifluoromethyl group, a linear or branched alkyl group having 1 to 6 carbon atoms which may have a substituent, a substituted or unsubstituted aromatic hydrocarbon group, a substituted or unsubstituted aromatic heterocyclic group, or a substituted or unsubstituted condensed polycyclic aromatic group; plurality of R₁ to R₉ present in one molecule may be the same or different from one another; and A₁ represents a trivalent group of a substituted or unsubstituted aromatic hydrocarbon, a trivalent group of a substituted or unsubstituted aromatic heterocycle, or a trivalent group of a substituted or unsubstituted condensed polycyclic aromatic).

4. The substituted pyridyl compound according to claim 1, represented by the following general formula (1-4): 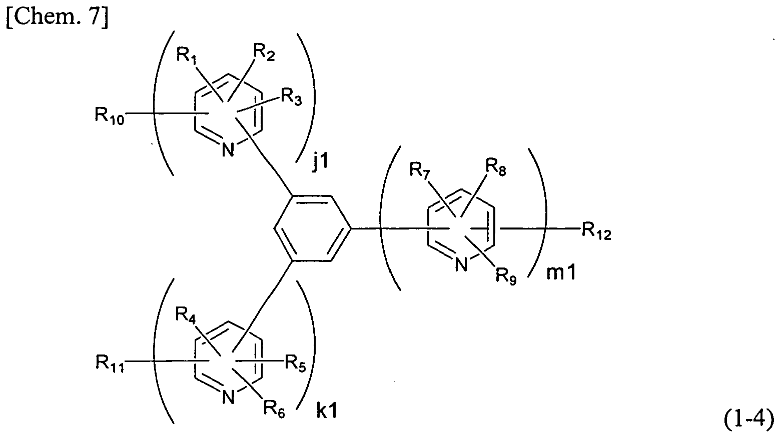 (wherein R₁ to R₁₂ may be the same or different and represent a hydrogen atom, a deuterium atom, a fluorine atom, a chlorine atom, a cyano group, a trifluoromethyl group, a linear or branched alkyl group having 1 to 6 carbon atoms which may have a substituent, a substituted or unsubstituted aromatic hydrocarbon group, a substituted or unsubstituted aromatic heterocyclic group, or a substituted or unsubstituted condensed polycyclic aromatic group; j1, k1, and m1 represent an integer of 2 or 3, excluding the case where all of j1, k1, and m1 have an identical value simultaneously; plurality of R₁ to R₉ present in one molecule may be the same or different from one another; and A₁ represents a trivalent group of a substituted or unsubstituted aromatic hydrocarbon, a trivalent group of a substituted or unsubstituted aromatic heterocycle, or a trivalent group of a substituted or unsubstituted condensed polycyclic aromatic).

5. The substituted pyridyl compound according to claim 1, represented by the following general formula (1-5): 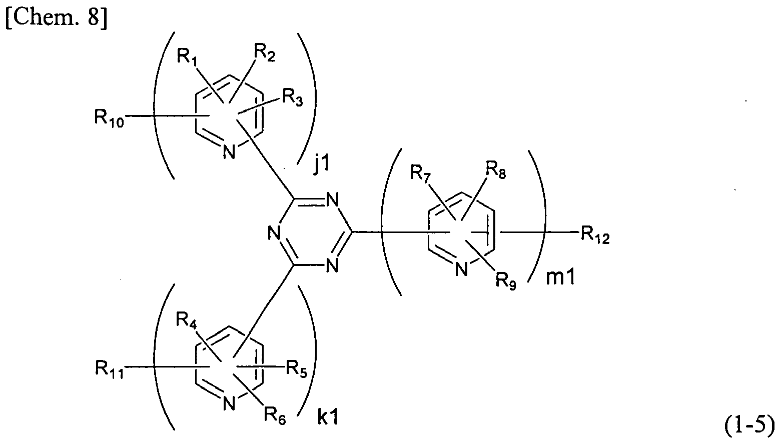 (wherein R₁ to R₁₂ may be the same or different and represent a hydrogen atom, a deuterium atom, a fluorine atom, a chlorine atom, a cyano group, a trifluoromethyl group, a linear or branched alkyl group having 1 to 6 carbon atoms which may have a substituent, a substituted or unsubstituted aromatic hydrocarbon group, a substituted or unsubstituted aromatic heterocyclic group, or a substituted or unsubstituted condensed polycyclic aromatic group; j1, k1, and m1 represent an integer of 2 or 3, excluding the case where all of j1, k1, and m1 have an identical value simultaneously; and plurality of R₁ to R₉ present in one molecule may be the same or different from one another).

6. The substituted pyridyl compound according to claim 1, represented by the following general formula (1-6): 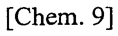 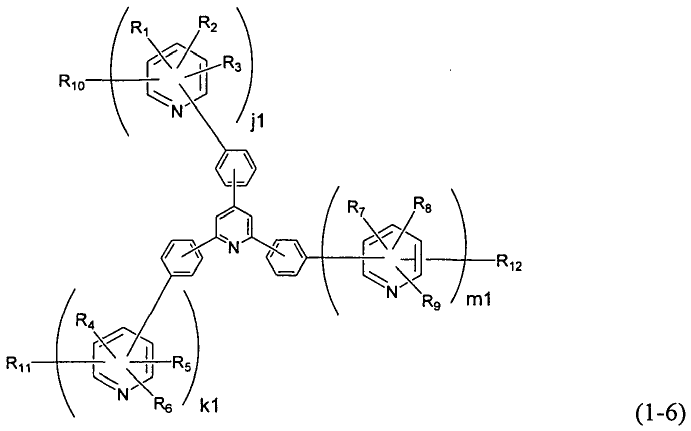 (wherein R₁ to R₁₂ may be the same or different and represent a hydrogen atom, a deuterium atom, a fluorine atom, a chlorine atom, a cyano group, a trifluoromethyl group, a linear or branched alkyl group having 1 to 6 carbon atoms which may have a substituent, a substituted or unsubstituted aromatic hydrocarbon group, a substituted or unsubstituted aromatic heterocyclic group, or a substituted or unsubstituted condensed polycyclic aromatic group; j1, k1, and m1 represent an integer of 2 or 3, excluding the case where all of j1, k1, and m1 have an identical value simultaneously; and plurality of R₁ to R₉ present in one molecule may be the same or different from one another).

7. An organic electroluminescent element comprising a pair of electrodes and at least one organic layer interposed therebetween, wherein the at least one organic layer contains the substituted pyridyl compound represented by the above general formula (1), (2), or (3).

8. The organic electroluminescent element according to claim 7, wherein the at least one organic layer contains an electron-transporting layer and the compound represented by the above general formula (1), (2), or (3) is present in the electron-transporting layer.

9. The organic electroluminescent element according to claim 7, wherein the at least one organic layer contains a hole-blocking layer and the compound represented by the above general formula (1), (2), or (3) is present in the hole-blocking layer.

10. The organic electroluminescent element according to claim 7, wherein the at least one organic layer contains an emitting layer and the compound represented by the above general formula (1), (2), or (3) is present in the emitting layer.

11. The organic electroluminescent element according to claim 7, wherein the at least one organic layer contains an electron-injecting layer and the compound represented by the above general formula (1), (2), or (3) is present in the electron-injecting layer.
